# EUROPÄISCHE PATENTSCHRIFT

(11) **EP 3 437 561 B1**
(45) Veröffentlichungstag und Bekanntmachung des Hinweises auf die Patenterteilung: **17.03.2021**
(21) Anmeldenummer: 17184262.8
(22) Anmeldetag: 01.08.2017
(51) Int. Cl.: A61B 6/04, A61B 6/00

(54) **WECHSELWIRKEN VON BETRIEBSPARAMETERN BEI EINEM MEDIZINISCHEN SYSTEM**
INTERACTING OF OPERATIVE PARAMETERS IN A MEDICAL SYSTEM
INTERACTION DE PARAMÈTRES DE FONCTIONNEMENT POUR UN SYSTÈME MÉDICAL

(43) Veröffentlichungstag der Anmeldung: 06.02.2019
(73) Patentinhaber: Siemens Healthcare GmbH, 91052 Erlangen (DE)
(72) Erfinder: Pickert, Nils, 91056 Erlangen (DE)

(56) Entgegenhaltungen:
- DE-A1-102012 215 998
- US-A1- 2006 109 954
- US-A1- 2010 037 394
- US-A1- 2016 113 615

## Beschreibung

Die vorliegende Erfindung betrifft ein Verfahren zum Betreiben eines medizinischen Systems, bei dem ein Patient gemäß einem Bewegungsparameter relativ zu einer Komponente des Systems bewegt wird. Darüber hinaus betrifft die vorliegende Erfindung ein medizinisches System mit einer Komponente und einer Bewegungseinrichtung, mit der ein Patient gemäß einem Bewegungsparameter relativ zu der Komponente bewegbar ist.

Aktuelle medizinische Röntgensysteme, die typischerweise aus einem Patiententisch und einem Röntgengerät bestehen, nutzen in der Regel feste Parametersätze für das Erstellen von Abbildungen durch das Röntgengerät und das Durchführen von Bewegungen des Patiententisches oder des Röntgengeräts. Die Parametersätze werden durch den Nutzer erstellt und während des Betriebs nicht mehr verändert. Gegebenenfalls werden für unterschiedliche Prozesse unterschiedliche Parametersätze eingesetzt.

Ein C-Bogen-Röntgengerät weist einige Abbildungsparameter auf. Darunter fallen beispielsweise die Dosis der Röntgenstrahlung, die Verstärkung der Detektorsignale, ein Zoomfaktor für die Abbildung und dergleichen. Vorrangiges Ziel bei der Durchleuchtung beziehungsweise Fluoroskopie ist es, die Röntgendosis für den Patienten, aber auch für das bedienende Personal, so gering wie möglich zu halten und eine sogenannte ALARA-Dosis (As low as is reasonably achievable) zu erreichen.

Ferner umfasst ein Durchleuchtungssystem in der Regel einen Tisch, auf dem der Patient relativ zu dem Röntgengerät bewegt werden kann. Für die verschiedenen Bewegungen können unterschiedliche Bewegungsparameter vorgesehen sein. Insbesondere kann der Tisch motorisch verstellbar sein. Gegebenenfalls ist er z.B. mittels eines Joysticks zumindest in einer Ebene frei bewegbar. Unter Umständen können die Bewegungen mit verschiedenen Geschwindigkeiten durchgeführt werden. Es sind aber auch Tische bekannt, die manuell in alle Richtungen bewegbar sind, sobald eine Bremse gelöst ist.

Bei medizinischen Systemen stehen im Betrieb üblicherweise auch Anatomiedaten zur Verfügung. Anatomiedaten können Bereiche des Körpers betreffen, die aktuell durchleuchtet werden. So können sich beispielsweise aktuelle Anatomiedaten auf das Herz beziehungsweise einen Herzbereich, auf das Rückgrat beziehungsweise einen Rückgratbereich oder auf Extremitäten oder einen Extremitätenbereich beziehen.

Aus der Druckschrift US 2016/113615 A1 sind ein radiographisches Abbildungsgerät und ein entsprechendes Steuerungsverfahren bekannt. Ein Patient wird relativ zu dem Röntgengerät bewegt. Ein Verhältnis zwischen der Bewegungsgeschwindigkeit und der Bestrahlung wird konstant gehalten.

Die Druckschrift DE 10 2012 215998 A1 offenbart ein Röntgengerät mit angepasster Aufnahmegeschwindigkeit. Patient und Aufnahmeeinrichtung bewegen sich relativ zueinander. Die Geschwindigkeit wird in Abhängigkeit von Röntgenabsorptionseigenschaften gesteuert.

Ferner beschreibt die Druckschrift US 2006/109954 A1 eine bildgebende Einheit, bei der eine Tischbewegung in dem Abbildungsraum erfolgt. Ein Bestrahlungsstrom wird in Abhängigkeit von der Tischgeschwindigkeit gesteuert.

Druckschrift US 2010/037394 A1 offenbart ein Verfahren zur Bewegungssteuerung während einer manuellen Patientenpositionierung. Dabei wird der Widerstand der manuellen Bewegung des Patiententisches in Abhängigkeit von einer Geschwindigkeit oder einer Position des Patiententisches gesteuert.

Die Aufgabe der vorliegenden Erfindung besteht darin, den Betrieb eines medizinischen Systems zu verbessern beziehungsweise zu vereinfachen.

Erfindungsgemäß wird diese Aufgabe gelöst durch ein Verfahren und ein medizinisches System gemäß den unabhängigen Ansprüchen. Vorteilhafte Weiterbildungen der Erfindung ergeben sich aus den Unteransprüchen.

Entsprechend der vorliegenden Erfindung wird demnach ein Verfahren zum Betreiben eines medizinischen Systems bereitgestellt, bei dem gemäß einem Bewegungsparameter ein Patient relativ zu einer Komponente des Systems bewegt wird. Das medizinische System umfasst ein bildgebendes System und einen Tisch, auf dem ein Patient platzierbar ist. Für die Untersuchung mit dem bildgebenden System kann der Patient auf dem Tisch relativ zu dem bildgebenden System bewegt werden. Die Bewegung des Patienten mithilfe des Tisches kann durch einen oder mehrere Bewegungsparameter charakterisiert werden.

Darüber hinaus wird das medizinische System meist durch einen oder mehrere weitere Prozessparameter im Betrieb bestimmt. Dieser eine Prozessparameter beziehungsweise die mehreren Prozessparameter sind verschieden von dem einen oder den mehreren Bewegungsparametern. Es liegen also ein Parameterpaar, -tripel und so weiter vor. Der Bewegungsparameter und in gleicher Weise der Prozessparameter können unterschiedliche Werte annehmen. Es sind aber in der Regel nicht sämtliche Kombinationen von Parameterwerten für den Betrieb sinnvoll. So ist es beispielsweise nicht sinnvoll, bei einem hohen Zoomfaktor eine hohe Geschwindigkeit zwischen den zueinander bewegten Komponenten zu erlauben. Ebenso wenig sinnvoll ist es, bei hohen Verfahrgeschwindigkeiten hohe Röntgendosen für hohe Bildqualität einzustellen. Daher sieht die vorliegende Erfindung ein automatisches Wechselwirken des Bewegungsparameters mit einem anderen Prozessparameter des medizinischen Systems vor, sodass sich ein Wert des Prozessparameters dynamisch mit einem Wert des Bewegungsparameters ändert. Es wird also ein Bewegungsparameter mit einem anderen Parameter des medizinischen Systems gekoppelt, der unabhängig von der Bewegung ist beziehungsweise diese nicht beeinflusst. Speziell können so Prozessparameter-Bewegungsparameter-Kombinationen vermieden werden, die nicht sinnvoll sind. Dabei ist vorgesehen, dass der Bewegungsparameter mit einem Sensor zur Messung der Geschwindigkeit erfasst wird und der Prozessparameter dynamisch angepasst wird.

Bei dem Bewegungsparameter handelt es sich um eine Z relative Geschwindigkeit des Patienten gegenüber einer als bildgebenden Einrichtung ausgebildeten Komponente.

Das medizinische System besitzt einen manuell verschiebbaren Tisch Der Prozessparameter ist ein Abbildungsparameter der Komponente, die als bildgebende Einrichtung ausgebildet ist. Der Prozessparameter betrifft also eine Abbildungseigenschaft der bildgebenden Einrichtung. Damit wird ein Abbildungsparameter des medizinischen Systems mit einem Bewegungsparameter des medizinischen Systems gekoppelt. Insbesondere kann so die Abbildungsqualität beziehungsweise - quantität von der Bewegung abhängig gemacht werden.

Entsprechend einer Ausführungsform handelt es sich bei dem Abbildungsparameter um eine Strahlungsintensität, eine Röntgendosis, einen Verstärkungsfaktor oder einen Zoomfaktor. So ist es beispielsweise notwendig beziehungsweise sinnvoll, die Strahlungsintensität beziehungsweise Röntgendosis zu reduzieren, wenn der Patient mit einer hohen Geschwindigkeit bewegt wird. Bei einer hohen Geschwindigkeit genügt es nämlich in der Regel, Bilder mit niedriger Auflösung zu gewinnen, um beispielsweise zu dem gewünschten Ort zu gelangen. Wird der Zielort erreicht, so wird üblicherweise auch die Geschwindigkeit reduziert (gegebenenfalls bis auf 0) und eine hohe Abbildungsqualität ist gewünscht. In diesem Fall sollte bei geringer Geschwindigkeit die Strahlungsintensität beziehungsweise die Röntgendosis wieder erhöht werden.

In einem anderen Beispiel kann ein hoher Zoomfaktor gewählt sein. In diesem Fall ist es nicht wünschenswert, wenn der Patient unter der bildgebenden Einrichtung rasch bewegt wird, denn durch die hohe Vergrößerung kann rasch die Orientierung verloren werden. Bei hoher Vergrößerung bewegen sich nämlich die Bilder entsprechend noch schneller auf dem Bildschirm.

Darüber hinaus kann vorgesehen sein, dass der Wert des Prozessparameters um einen festen Wert verändert wird, wenn der Wert des Bewegungsparameters einen vorbestimmten Wert überschreitet. Wenn die Geschwindigkeit, mit der der Patient relativ zu der Komponente des medizinischen Systems bewegt wird, größer ist als eine vorbestimmte Geschwindigkeit, so wird beispielsweise der Prozessparameter um eine Stufe herabgesetzt, halbiert oder anderweitig stufenweise verändert. So kann es beispielsweise günstig sein, den Zoomfaktor auf die kleinste Stufe zu stellen, wenn eine gewisse Geschwindigkeit des Patiententisches überschritten wird.

In einer Weiterentwicklung kann vorgesehen sein, dass der Wert des Prozessparameters kontinuierlich mit dem Bewegungsparameter verändert wird. Der Wert des Prozessparameters wird hier also nicht stufenweise, sondern stufenlos beziehungsweise kontinuierlich verändert. So kann beispielsweise ein linearer, ein quadratischer, ein exponentieller oder ein anderer stetiger Zusammenhang zwischen dem Prozessparameter und dem Bewegungsparameter bestehen.

Die zuvor im Zusammenhang mit dem erfindungsgemäßen Verfahren erwähnten Vorteile und Weiterbildungsmöglichkeiten können sinngemäß auch auf das medizinische System übertragen werden. Dabei gelten die einzelnen Verfahrensschritte als funktionelle Merkmale geeigneter Komponenten des medizinischen Systems (z.B. Bewegungseinrichtung, Steuerungseinrichtung und dergleichen) .

In einer speziellen Ausgestaltung kann die Komponente ein Röntgengerät und insbesondere ein C-Bogen-Röntgengerät sein. Es wird also der Patient relativ zu dem Röntgengerät bewegt. Beispielsweise handelt es sich bei der Bewegung um eine Angulation des C-Bogen-Röntgengeräts. Ein diesbezüglicher Parameter wird nun abhängig gemacht von einem anderen Prozessparameter, der insbesondere ein Abbildungsparameter des Röntgengeräts ist.

In einer weiteren Ausführungsform, die nicht Bestandteil der Erfindung ist, kann vorgesehen sein, dass das medizinische System einen Tisch aufweist, auf dem der Patient manuell oder motorisch bewegbar ist. Insbesondere kann es sich bei dem Tisch um einen Operationstisch handeln. Falls der Patient beziehungsweise der Operationstisch manuell verschiebbar ist, kann die Reibung einer Reibungsbremse automatisch erhöht werden, wenn der Zoomfaktor des bildgebenden Röntgengeräts hoch ist. So kann beispielsweise die Reibung linear mit dem Zoomfaktor erhöht werden. Beim manuellen Anschieben des Tisches muss der Bediener dann einen höheren Widerstand überwinden, um den Tisch zu bewegen.

Die vorliegende Erfindung wird nun anhand der beigefügten Zeichnungen näher erläutert, in denen zeigen:
- FIG 1: ein Ablaufdiagramm eines erfindungsgemäßen Verfahrens gemäß einem ersten Ausführungsbeispiel,
- FIG 2: ein Ablaufdiagramm eines Verfahrens gemäß einem zweiten Ausführungsbeispiel, das nicht Bestandteil der Erfindung ist, und
- FIG 3: eine Prinzipskizze eines erfindungsgemäßen Röntgensystems.

Die nachfolgend näher geschilderten Ausführungsbeispiele stellen bevorzugte Ausführungsformen der vorliegenden Erfindung dar. Dabei ist zu beachten, dass die einzelnen Merkmale nicht nur in ihren geschilderten Merkmalskombinationen, sondern auch in Alleinstellung oder in anderen technisch sinnvollen Kombinationen realisiert werden können.

Die Grundidee basiert darauf, dass ein Betriebsparameter während des Betriebs angepasst wird, um eine verbesserte Nutzung zu erreichen und insbesondere bei bildgebenden medizinischen Geräten die Strahlungsdosis so gering wie möglich zu halten.

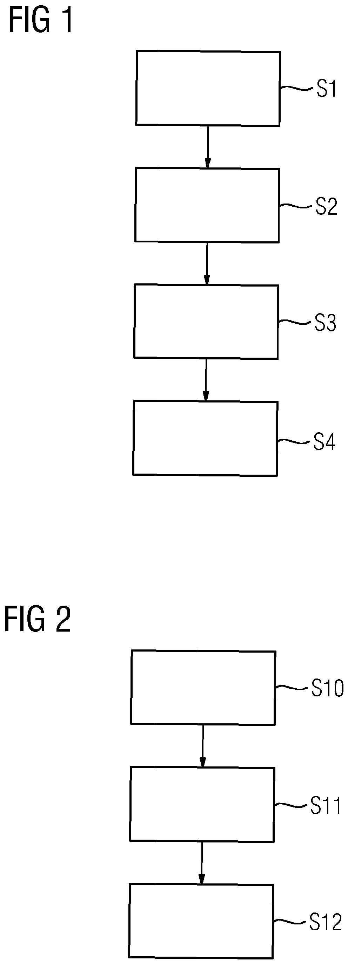

In dem Beispiel von FIG 1 wird die Strahlungsdosis während einer Bewegung des Patienten relativ zu dem Aufnahmegerät, das heißt einer Komponente des medizinischen Systems, reduziert. Speziell werden beispielsweise die Röntgenparameter in einem Angioskopiesystem für optimale Bildqualität bei einer ALARA-Dosis für ein nichtbewegendes System gemäß Schritt S1 fest eingestellt. Nun wird beispielsweise der Tisch, auf dem sich der Patient befindet, oder der C-Bogen des Röntgengeräts bei einer Aufnahme beziehungsweise Durchleuchtung entsprechend Schritt S2 bewegt. Mithin bewegt sich der Patient relativ zu einer Komponente des medizinischen Systems beziehungsweise des Röntgengeräts. Üblicherweise würde während der Bewegung die Bildqualität unverändert hoch beibelassen werden, obwohl dies aus Nutzersicht nicht notwendig ist. Es wäre akzeptabel, die Dosis während der Bewegung zu reduzieren (und anschließend wieder zu erhöhen), da das menschliche Auge die volle Bildqualität während der Bewegung meist nicht auflösen kann.

Das Bewegen des Tisches gemäß Schritt S2 erfolgt manuell. Bei motorischer Bewegung ist in der Regel unmittelbar auch die Geschwindigkeit des Tisches bekannt. Ein entsprechender Geschwindigkeitswert des Bewegungsparameters "Geschwindigkeit" kann vom System bereitgestellt werden.

Da der Tisch jedoch manuell bewegt wird, liegt die Geschwindigkeit des Tisches nicht unmittelbar vor. Vielmehr muss sie entsprechend Schritt S3 gemessen werden. Ein entsprechender Sensor in dem medizinischen System erfasst also die relative Geschwindigkeit des Patienten gegenüber einer Komponente des medizinischen Systems (z.B. Röntgendetektor). In einem anschließenden Schritt S4 erfolgt ein Zuordnen beziehungsweise Einstellen der Dosis in Abhängigkeit von dem Bewegungsparameter. Das Zuordnen beziehungsweise Einstellen kann in Stufen oder stufenlos erfolgen. Beispielsweise wird die Strahlungsdosis umso weiter reduziert, je höher die detektierte Geschwindigkeit zwischen Patienten und Aufnahmegerät ist.

Beispielsweise erfolgt eine automatische Dosisreduktion während einer Bildaufnahme beziehungsweise Durchleuchtung, wenn der Patiententisch oder der C-Bogen in eine andere Position gebracht wird. Daraus resultiert eine etwas geringere Bildqualität während der Bewegung. Gleichzeitig wird aber auch die Dosis auf den Patienten und das Personal reduziert, ohne dass die medizinischen Ergebnisse beeinträchtigt werden.

Das Einstellen einer verminderten Dosis beziehungsweise die Erhöhung der Detektorverstärkung, wenn das System bewegt wird, reduziert die Gesamtbelastung des Patienten während der Durchleuchtung. Dabei kann die Dosisreduktion allein dadurch erreicht werden, dass die Strahlung um einen festen Faktor beziehungsweise Betrag herabgesetzt wird. Beispielsweise wird die Strahlungsdosis bei einer Bewegung des Systems automatisch auf die Hälfte herabgesetzt. Darüber hinaus ist es aber auch möglich, den Dosisreduktionsfaktor unmittelbar abhängig von dem Geschwindigkeitswert der Bewegung zu wählen. Je schneller die Bewegung ist, desto weniger Details können durch das menschliche Auge aufgelöst werden, und desto mehr Dosisreduktion kann erreicht werden. Beispielsweise kann so bei Angiographieprozeduren eine deutlich niedrigere Dosis erreicht werden als bei bekannten Systemen.

In einem nicht beanspruchten Beispiel gemäß FIG 2 erfolgt eine Anpassung der Bewegungsgeschwindigkeit beziehungsweise der Systemrückkopplung in Abhängigkeit von Bildgebungseinstellungen. Bei üblichen Systemen aus dem Stand der Technik wird eine Systembewegungsgeschwindigkeit oder eine Kraft für eine manuelle Bewegung et cetera unabhängig von anderen Einstellungen festgelegt. Ein bekanntes Problem ist beispielsweise die präzise Positionierung des Tisches oder des C-Bogens eines Angiographiesystems während eines Röntgenvorgangs, wenn ein hoher Zoomfaktor eingestellt ist. Eine geeignete Einstellung hängt viel von der Erfahrung und der Kenntnis des Nutzers ab. Daher wird erfindungsgemäß vorgeschlagen, die Kräfte, die zum manuellen Bewegen des Systems notwendig sind, oder die Geschwindigkeit einer motorisierten Bewegung des Systems auf der Basis z.B. der Zoomfaktoreinstellung oder eines anderen Parameters anzupassen. Ein derartiger anderer Parameter kann beispielsweise die SID (Source-Image-Distance) oder die Angulation sein.

Die Kraft, mit der ein Tisch manuell bewegt werden kann, kann durch ein automatisches Einstellen der Reibungskraft einer am Tisch verbauten Bremse erreicht werden. Die Anpassung der Geschwindigkeit einer motorisierten Bewegung kann durch die entsprechende Ansteuerung des eingesetzten Motors erzielt werden. Durch die Anpassung der Geschwindigkeit erhält man eine ausgewählte Sensitivität, die es erlaubt, das System präziser zu positionieren.

Konkret wird also beispielsweise gemäß Schritt S10 ein Zoomfaktor eingestellt, wie dies in FIG 2 schematisch in einem Ablaufdiagramm dargestellt ist. In einem anschließenden Schritt S11 erfolgt beispielsweise ein Zuordnen des Zoomfaktors zu einer Maximalgeschwindigkeit oder einer Reibung, wobei letztere die Beschleunigung beeinflusst. Schließlich wird der Tisch oder eine andere Komponente des medizinischen Systems gemäß Schritt S12 mit einer Geschwindigkeit bewegt, die die Maximalgeschwindigkeit nicht übersteigt.

Konkret kann also abhängig von einem Prozessparameter wie dem Zoomfaktor, SID (d.h. Vergrößerung), Angulation oder Anatomiedaten in einem medizinischen System die Empfindlichkeit der Bewegung durch Verlangsamen oder Beschleunigen der motorisierten Bewegungen und/oder durch Einstellen der Reibung für manuelle Bewegungen adaptiert werden. Wenn sich also beispielsweise die Bildgebung auf einen anatomischen Bereich oder einen speziellen Prozess bezieht, der eine feinere Justage erfordert (z.B. Neuro- oder Wirbelsäulenchirurgie), so kann dies automatisch über die entsprechenden Prozessparameter erreicht werden. Auf diese Weise kann eine schwimmend gelagerte Tischplatte eines OP-Tisches sehr präzise bei einer Röntgenuntersuchung platziert werden.

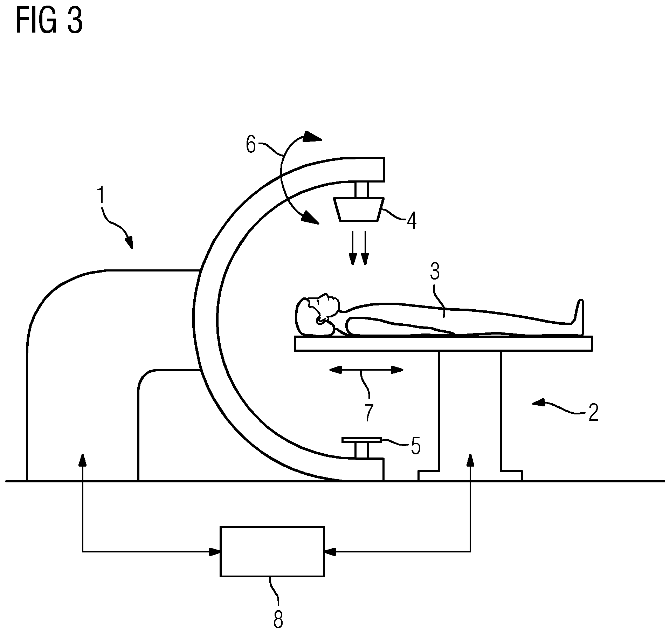

FIG 3 zeigt schematisch ein medizinisches System mit einem C-Bogen-Röntgengerät 1 und einem Patiententisch 2, auf dem sich ein Patient 3 befindet. Das C-Bogen-Röntgengerät 1 besitzt an den distalen Enden des C-Bogens eine Röntgenstrahlungsquelle 4 und gegenüber einen Detektor 5. Der C-Bogen ist durch Angulation 6 relativ zu dem Patienten 3 bewegbar beziehungsweise schwenkbar. Darüber hinaus kann der C-Bogen noch weitere Bewegungsfreiheitsgrade besitzen. Auch der Patiententisch 2 erlaubt Tischbewegungen 7 hauptsächlich in einer Ebene, aber auch senkrecht dazu.

Das medizinische System gemäß FIG 3 besitzt also eine Komponente (z.B. Detektor 5), gegenüber der der Patient 3 mittels dem Patiententisch mit einem Bewegungsparameter relativ zu der Komponente bewegbar ist. Außerdem verfügt das medizinische System über eine Steuerungseinrichtung 8, die für eine Wechselwirkungsmöglichkeit des Bewegungsparameters mit einem anderen Prozessparameter des medizinischen Geräts sorgt. Insbesondere kann sich ein Wert des Prozessparameters (z.B. Abbildungsparameter wie Röntgendosis) dynamisch mit einem Wert des Bewegungsparameters (z.B. Bewegungsgeschwindigkeit des Patiententisches 2) ändern. Die Steuereinrichtung kann auch beispielsweise in das C-Bogen-Röntgengerät 1 oder den Patiententisch 2 integriert sein.

In einem konkreten Beispiel wird in den Patienten 3 ein Katheder eingeschoben. Während des Einschiebens wird der Patient in seiner Position derart nachgeführt, dass die Kathederspitze stets im Aufnahmebereich des Detektors 5 liegt. Da sich der Patiententisch 2 bewegt, wird während dieser Bewegung die Strahlendosis um einen festen Wert oder einen mit der Geschwindigkeit korrespondierenden Wert herabgesetzt. Auf diese Weise kann beispielsweise die Gesamtdosis für den Patienten während der Operation auf 80 Prozent oder einen anderen Wert reduziert werden.

## Patentansprüche

1. Verfahren zum Betreiben eines medizinischen Systems, bei dem ein Patient (3) manuell mittels einem verschiebbaren Patiententisch (2) relativ zu einer als bildgebende Einrichtung ausgebildeten Komponente (1, 4, 5) des Systems mit einem Bewegungsparameter bewegt wird (S2, S12), umfassend die folgenden Schritte: Erfassen einer relativen Geschwindigkeit des Patienten (3) gegenüber der Komponente (1, 4, 5) als der Bewegungsparameter mit einem Sensor (S3), und automatisches Wechselwirken (S4) eines Abbildungsparameters der bildgebenden Einrichtung mit dem Bewegungsparameter, sodass sich der Wert des Abbildungsparameters dynamisch mit dem Wert des Bewegungsparameters ändert.

2. Verfahren nach Anspruch 1, wobei der Abbildungsparameter eine Strahlungsintensität, eine Röntgendosis, ein Verstärkungsfaktor oder ein Zoomfaktor ist.

3. Verfahren nach einem der Ansprüche 1 bis Z 2, wobei der Wert des Abbildungsparameters um einen festen Wert verändert wird, wenn der Wert des Bewegungsparameters einen vorbestimmten Wert überschreitet.

4. Verfahren nach einem der Ansprüche 1 bis 2, wobei der Wert des Abbildungsparameters kontinuierlich mit dem Bewegungsparameter verändert wird.

5. Medizinisches System mit
- einer Komponente (1, 4, 5), die als bildgebende Einrichtung ausgebildet ist,
- einem verstellbaren Patiententisch (2), mit dem ein Patient (3) relativ zu der Komponente manuell mit einem Bewegungsparameter bewegbar ist,
- einem Sensor zum Erfassen einer relativen Geschwindigkeit des Patienten (3) gegenüber der Komponente (1, 4, 5) als der Bewegungsparameter, und mit
- einer Steuerungseinrichtung (8), mittels derer ein automatisches Wechselwirken eines Abbildungsparameters der bildgebenden Einrichtung mit dem Bewegungsparameter erfolgt, sodass sich der Wert des Abbildungsparameters dynamisch mit dem Wert des Bewegungsparameters ändert.

6. Medizinisches System nach Anspruch 5, wobei die Komponente (1, 4, 5) ein Röntgengerät und insbesondere ein C-Bogen-Röntgengerät (1) ist.

## Claims

1. Method for operating a medical system, in which a patient (3) is moved manually by means of a patient table (2) that can be moved relative to a component (1, 4, 5), which is embodied as an imaging apparatus, of the system with a motion parameter, comprising the following steps: detection of a speed of the patient (3) relative to the component (1, 4, 5) as the motion parameter using a sensor (S3), and automatic interaction (S4) between an image parameter of the imaging apparatus and the motion parameter so that the value of the image parameter changes dynamically with the value of the motion parameter.

2. Method according to claim 1, wherein the image parameter is a radiation intensity, an X-ray dose, a gain factor or a zoom factor.

3. Method according to one of claims 1 to 2, wherein the value of the image parameter is altered by a fixed value if the value of the motion parameter exceeds a predetermined value.

4. Method according to one of claims 1 to 2, wherein the value of the image parameter is altered continuously with the motion parameter.

5. Medical system comprising
- a component (1, 4, 5), which is embodied as an imaging apparatus,
- an adjustable patient table (2) that can be used to manually move a patient (3) relative to the component with a motion parameter,
- a sensor for detecting a speed of the patient (3) relative to the component (1, 4, 5) as the motion parameter, and comprising
- a controller (8), by means of which an automatic interaction takes place between an image parameter of the imaging apparatus and the motion parameter so that the value of the image parameter changes dynamically with the value of the motion parameter.

6. Medical system according to claim 5, wherein the component (1, 4, 5) is an X-ray device and in particular a C-arm X-ray device (1).

## Revendications

1. Procédé pour faire fonctionner un système médical dans lequel on déplace (S2, S12), avec un paramètre de déplacement, un patient (3) manuellement au moyen d'une table (2) de patient déplaçable par rapport à un élément (1, 4, 5), constitué en dispositif d'imagerie, comprenant les stades suivants :
On relève par un capteur (S3) une vitesse relative du patient (3) par rapport à l'élément (1, 4, 5) comme paramètre de déplacement et on met (S4) en interaction automatique un paramètre de représentation du dispositif d'imagerie avec le paramètre de déplacement de manière à modifier la valeur du paramètre de représentation dynamiquement avec la valeur du paramètre de déplacement.

2. Procédé suivant la revendication 1, dans lequel le paramètre de représentation est une intensité de rayonnement, une dose de rayons X, un facteur d'amplification ou un facteur de zoom.

3. Procédé suivant l'une des revendications 1 à 2, dans lequel on modifie la valeur du paramètre de représentation d'une valeur fixe si la valeur du paramètre de déplacement dépasse une valeur déterminée à l'avance.

4. Procédé suivant l'une des revendications 1 à 2, dans lequel on modifie la valeur du paramètre de représentation continuellement avec le paramètre de déplacement.

5. Système médical comprenant
- un élément (1, 4, 5) qui est constitué sous la forme d'un dispositif d'imagerie,
- une table (2) de patient déplaçable, par laquelle un patient (3) peut être déplacé avec un paramètre de déplacement manuellement par rapport à l'élément,
- un capteur pour relever une vitesse relative du patient (3) par rapport à l'élément (1, 4, 5) comme paramètre de déplacement et comprenant
- un dispositif (8) de commande, au moyen duquel a lieu une interaction automatique d'un paramètre de représentation du dispositif d'imagerie avec le paramètre de déplacement de manière à modifier la valeur du paramètre de représentation dynamiquement avec la valeur du paramètre de déplacement.

6. Système médical suivant la revendication 5, dans lequel l'élément (1, 4, 5) est un appareil à rayons X et notamment un appareil (1) à rayons X à arceau en C.
